# EUROPEAN PATENT SPECIFICATION

(11) **EP 1 380 301 B1**
(45) Date of publication and mention of the grant of the patent: **14.01.2009**
(21) Application number: 03017743.0
(22) Date of filing: 31.08.2000
(51) Int. Cl.: A61K 31/565, A61K 31/585, A61P 15/00

(54) **Pharmaceutical combination of ethinylestradiol and dropirenone for use as a contraceptive**
Pharmazeutische Kombination von Ethinylestradiol und Drospirenon als empfängnisverhütendes Mittel
Combinaison pharmaceutique d'éthinylestradiol et de drospirénone comme contraceptif

(30) Priority: 31.08.1999 EP 99202826; 31.08.1999 US 386274
(43) Date of publication of application: 14.01.2004
(62) Divisional of application: 00953387.8
(73) Proprietor: Bayer Schering Pharma Aktiengesellschaft, 13353 Berlin (DE)
(72) Inventor: Heil, Wolfgang, 21453 Stelle (DE); Hilman, Jürgen, 13351 Berlin (DE); Lipp, Ralph, 14169 Berlin (DE); Heithecker, Renate, 14163 Berlin (DE)
(74) Representative: Plougmann & Vingtoft A/S

(56) References cited:
- WO-A-01/52857
- WO-A-95/17194
- WO-A-95/26730
- WO-A-97/01342
- WO-A-98/04267
- WO-A-98/04269
- NUERNBERG E.: "[Manufacturing and properties of spray dried products]. HERSTELLUNG UND EIGENSCHAFTEN PHARMAZEUTISCHER SPRUHTROCKNUNGSPRODUKTE." ACTA PHARMACEUTICA HUNGARICA, (1978) 48/1 (19-35). CODEN: APHGAO, XP000908820
- OELKERS W K H: "Effects of estrogens and progestogens on the renin-aldosterone system and blood pressure" STEROIDS: STRUCTURE, FUNCTION, AND REGULATION,US,ELSEVIER SCIENCE PUBLISHERS, NEW YORK, NY, vol. 61, no. 4, 1 April 1996 (1996-04-01), pages 166-171, XP004026568 ISSN: 0039-128X
- OELKERS W ET AL: "EFFECTS OF A NEW ORAL CONTRACEPTIVE CONTAINING AN ANTIMINERALOCORTICOID PROGESTOGEN, DROSPIRENONE, ON THE RENIN-ALDOSTERONE SYSTEM, BODY WEIGHT, BLOOD PRESSURE, GLUCOSE TOLERANCE, AND LIPID METABOLISM" JOURNAL OF CLINICAL ENDOCRINOLOGY AND METABOLISM,US,NEW YORK, NY, vol. 80, no. 6, 1 June 1995 (1995-06-01), pages 1816-1821, XP002046619 ISSN: 0021-972X
- MUHN P ET AL: "Drospirenone: a novel progestogen with antimineralocorticoid and antiandrogenic activity. Pharmacological characterization in animal models." CONTRACEPTION, (1995 FEB) 51 (2) 99-110., XP000908817
- KINCL: "increasing oral bioavailability of progesterone by formulation" JOURNAL OF STEROID BIOCHEMISTRY, vol. 9, - 1978 pages 83-84,

## Description

### FIELD OF THE INVENTION

The present invention relates to a pharmaceutical composition comprising drospirenone and ethinylestradiol and the use of drospirenone and ethinylestradiol for inhibiting ovulation.

### BACKGROUND OF THE INVENTION

Oral contraceptives containing a combination of a gestagen and an estrogen component have been used since the 1960's. The earliest contraceptive preparations consisted of 21 tablets containing the combination of active agents and 7 tablets containing no active agent, and the amount of each active agent was the same in each tablet (the so-called one-phase preparations). Subsequently, preparations were developed that consisted of tablets containing different amounts and ratios of the active agents over the cycle of administration (the so-called multiple-phase preparations).

Contraceptive reliability is mainly provided by the gestagen component. The daily dosage should be at least the minimum of what is needed for the gestagen in question to inhibit ovulation effectively. The estrogen component acts to increase the ovulation inhibitory effect of gestagen and to ensure cycle stability. Since the introduction of oral contraceptives, the daily dosage of gestagen has been reduced through the development of new and more efficient gestagens than were present in the earlier contraceptive preparations. It has also been possible to reduce the daily dosage of estrogen.

6β,7β;15β,16β-dimethylene-3-oxo-17α-pregn-4-ene-21,17-carbolactone (drospirenone) is known from DE 26 52 761 in which its use as a diuretic compound is disclosed.

In DE 30 22 337, the gestagen-like activity of the compound and its consequent utility as a contraceptive agent is described at dosage levels of 0.5-50 mg of drospirenone per day. It is also noted that the mechanism of action of the compound is very similar to that of the natural corpus luteum hormone progesterone, and that it does not give rise to increased blood pressure for which reason it may be administrated to women who have or are at risk of developing increased blood pressure. It is further described that drospirenone may be administered together with ethinylestradiol in an amount of 0.03-0.05 mg per day.

DE 30 51 166 discloses the use of the drospirenone for the treatment of gynaecological irregularities and for contraception at a dosage level of 0.5-50 mg per day.

EP 398 460 discloses the use of drospirenone for the treatment of androgen-induced disorders, aldosterone-induced disorders and hormonal irregularities as well as for contraception at dosage levels of 0.5-50 mg, preferably 1-10 mg per day. Ethinylestradiol may be co-administered at a level of 0.02-0.04 mg per day.

US 5,756,490 discloses pharmaceutical combination preparations comprising 23 or 24 dosage units containing a combination of a gestagen and an estrogen and 4-10 dosage units containing estrogen alone. Drospirenone is mentioned as a possible, but not preferred, gestagenic compound and ethinylestradiol is mentioned as a possible, but not preferred, estrogenic compound.

WO 98/04267 discloses oral contraceptives containing 0.25-4 mg drospirenone and 10-20 µg ethinylestradiol. These doses are administered for 23-25 days beginning on day one of the menstrual cycle, and is followed by plain ethinylestradiol dosing (5-15 µg) for the last 3-5 days of the menstrual cycle. WO 98/04267 does not disclose compositions having a rapid dissolution of drospirenone.

Oelkers et al. (Journal of Clinical Endocrinology and Metabolism, 1995;80; 1816-1821) investigated the effect of a combination of ethinylestradiol and drospirenone on, for example, body weight and blood pressure. Oelkers et al. does not disclose compositions having a rapid dissolution of drospirenone.

Kincl et al. (Journal of Steroid Biochemistry, 1978;9;83-84) reported that the poor bioavailability of progesterone can be partially overcome by addition of a cholesterol ester. Drospirenone is not mentioned by Kincl. et al.

### SUMMARY OF THE INVENTION

In a first aspect the present invention relates to a pharmaceutical composition comprising, as a first active agent drospirenone in an amount corresponding to a daily dosage, on administration of the composition, of from about 2 mg to 4 mg, and as a second active agent, ethinylestradiol in an amount corresponding to a daily dosage of from about 0.01 mg to 0.05 mg, together with one or more pharmaceutically acceptable carriers or excipients, wherein at least 70% of said drospirenone is dissolved from said composition within 30 minutes, as determined by USP XXIII Paddle Method II using water at 37°C as the dissolution media and 50 rpm as the stirring rate.

In another aspect the present invention relates to a pharmaceutical preparation consisting of a number of separately packaged and individually removable daily dosage units placed in a packaging unit and intended for oral administration for a period of at least 21 consecutive days, wherein said daily dosage units comprises a combination of drospirenone in an amount of from about 2 mg to 4 mg and ethinylestradiol in an amount from about 0.01 to 0.05 mg, wherein at least 70% of said drospirenone is dissolved from said dosage unit within 30 minutes, as determined by USP XXIII Paddle Method II using water at 37°C as the dissolution media and 50 rpm as the stirring rate.

In a further aspect the present invention relates to the use of drospirenone combined with ethinylestradiol for preparing a pharmaceutical composition for the inhibition of ovulation in a mammal, in particular a human, the composition comprising an amount of drospirenone corresponding to a daily dosage, on administration of the composition, of from about 2 mg to 4 mg, and comprising an amount of ethinylestradiol corresponding to a daily dosage, on administration of the composition, of from about 0.01 to 0.05 mg, wherein at least 70% of said drospirenone is dissolved from said composition within 30 minutes, as determined by USP XXIII Paddle Method II using water at 37°C as the dissolution media and 50 rpm as the stirring rate.

### BRIEF DESCRIPTION OF THE DRAWINGS

The invention is further described with reference to the drawings in which
Fig. 1 is a graph showing the in vitro dissolution rate of drospirenone from tablet cores, V1-V7 being batches containing micronized drospirenone, and V8 being a batch containing macrocrystalline drospirenone;
Fig. 2 is a graph showing the in vitro dissolution rate of drospirenone from tablet cores, different lines representing different test batches;
Fig. 3 is a graph showing the in vitro dissolution rate of drospirenone from film-coated tablets, different lines representing different test batches;
Fig. 4 is a graph showing the in vitro dissolution rate of ethinylestradiol from tablet cores, different lines representing different test batches; and
Fig. 5 is a graph showing the in vitro dissolution rate of ethinylestradiol from film-coated tablets, different lines representing different test batches.

### DETAILED DISCLOSURE OF THE INVENTION

Drospirenone, which may be prepared substantially as described in, e.g., US 4,129,564 or WO 98/06738, is a sparingly soluble substance in water and aqueous buffers at various pH values. Furthermore, drospirenone is rearranged to an inactive isomer under acid conditions and hydrolysed under alkaline conditions. To ensure good bioavailability of the compound, it is therefore advantageously provided in a form that promotes rapid dissolution thereof.

It has surprisingly been found that when drospirenone is provided in a pharmaceutical composition allowing for rapid dissolution of drospirenone a high oral bioavailability of drospirenone is obtained ("rapid dissolution" is defined as the dissolution of at least 70% within 30 minutes, in particular at least 80% within 20 minutes, of drospirenone from the composition as determined by the USP XXIII Paddle Method using a USP dissolution test apparatus 2 at 50 rpm and using water at 37°C as the dissolution media). This may be achieved by providing drospirenone in micronized form, or by dissolving it in a suitable solvent, e.g. methanol or ethyl acetate, and spray it onto the surface of inert carrier particles followed by incorporation of the particles containing drospirenone on their surface in the composition.

Without wishing to be limited to any particular theory, it appears that the in vitro dissolution rate of drospirenone is connected to the dissolution rate in vivo resulting in rapid absorption of drospirenone in vivo on oral administration of the compound. This is an advantage because isomerization of the compound in the gastric environment and/or hydrolysis in the intestine is substantially reduced, leading to a high bioavailability of the compound.

With respect to ethinylestradiol which is also a sparingly soluble substance, though less sensitive to degradation than drospirenone under conditions prevailing in the gastrointestinal tract, it is an advantage to provide it in micronized form or sprayed from a solution, e.g. in ethanol, onto the surface of inert carrier particles. This has the added advantage of facilitating a more homogenous distribution of the ethinylestradiol throughout the composition which might otherwise be difficult to obtain because ethinylestradiol is incorporated in extremely small amounts. When ethinylestradiol is provided in micronized form, it preferably has the following particle size distribution as determined under the microscope: 100% of the particles have a diameter of ≤ 15.0 µm, 99% of the particles have a diameter of ≤ 12.5µm, 95% of the particles have a diameter of ≤ 10.0 µm, and 50% of the particles have a diameter of ≤ 3.0 µm. Furthermore, no particle is larger than 20 µm, and ≤ 10 particles have a diameter of ≥ 15 µm and ≤ 20 µm.

To obtain a more rapid rate of dissolution, it is preferred to include carriers or excipients which act to promote dissolution of both active substances. Examples of such carriers and excipients include substances that are readily soluble in water such as cellulose derivatives, carboxymethylcellulose, hydroxypropyl cellulose, hydroxypropylmethyl cellulose, gelled starch, gelatin or polyvinylpyrrolidone. In particular, it appears as though polyvinylpyrrolidone might be particularly helpful to promote dissolution.

The composition of the invention preferably comprises drospirenone in an amount corresponding to a daily dosage of from 2.5 mg to 3.5 mg, in particular about 3 mg. The amount of ethinylestradiol preferably corresponds to a daily dosage of from 0.015 mg to 0.04 mg, in particular from 0.015 mg to 0.03 mg. More particularly, the present composition comprises an amount of drospirenone corresponding to a daily dosage of from 3.0 to 3.5 mg and ethinylestradiol in an amount corresponding to from 0.015 to 0.03 mg.

Apart from its ability to inhibit ovulation, the composition of the invention has been found to possess pronounced anti-androgenic properties. Furthermore, since drospirenone is an aldosterone antagonist, it has diuretic properties and is therefore suitable for counteracting the water-retentive properties of ethinylestradiol.

In a particular embodiment, the invention relates to a pharmaceutical preparation consisting of a number of separately packaged and individually removable daily dosage units placed in a packaging unit and intended for oral administration for a period of at least 21 consecutive days, wherein each of said daily dosage units comprises a combination of drospirenone in an amount of from 2 mg to 4 mg and ethinylestradiol in an amount from 0.01 to 0.05 mg, and wherein at least 70% of said drospirenone is dissolved from said dosage unit within 30 minutes, as determined by USP XXIII Paddle Method II using media and 50 rpm as the stirring rate.

In one embodiment, the preparation further comprises 7 or less said daily dosage units containing no active agent. Alternatively, it is possible to include, in the dosage regimen, a period of 7 days or less during which no dosage units are ingested. For compliance reasons, however, it may be preferred to include an appropriate number of blanks in the preparation, in which case the total number of daily dosage units in the preparation is at least 28. The inclusion of blanks, or the pill-free days, will then trigger withdrawal bleeding.

The preparation may be a one-phase composition, i.e. a preparation wherein the amounts of either active agent remains constant for the entire at least 21-day period, or the amounts of either or both active agents may be varied over the at least 21-day period to generate a multiple-phase preparation, e.g. a two- or three-phase preparation, substantially as disclosed in, e.g., EP 148 724.

In suitable embodiments of the present preparation, the number of daily dosage units comprising the combination of drospirenone and ethinylestradiol may be 21, 22, 23 or 24, and the number of daily dosage units containing no active agent may then be 7, 6, 5 or 4, as the case may be. In a further embodiment of the present preparation, the number of daily dosage units comprising the combination of drospirenone and ethinylestradiol is 28, or a multiple of 28 such as 2-4, in particular 2 or 3, times 28.

In an alternative embodiment, the invention relates to a contraceptive preparation consisting of a number of separately packaged and individually removable daily dosage units placed in a packaging unit and intended for oral administration for a period of at least 28 consecutive days, wherein at least 21 of said daily dosage units comprises a combination of drospirenone in an amount of from 2 mg to 4 mg and ethinylestradiol in an amount from 0.01 to 0.05 mg, and wherein 7 or less of said daily dosage units contain ethinylestradiol alone in an amount from 0.01 to 0.05 mg.

By including an appropriate number of dosage units comprising ethinylestradiol alone, high contraceptive reliability, low follicular development and satisfactory cycle control with little or no intermenstrual bleeding may be obtained.

In this case, too, the preparation may be one in which the amounts of either active agent remains constant for the entire at least 21-day period (i.e. a two-phase preparation), or the amounts of either or both active agents may be varied over the at least 21-day period to generate a multiple-phase preparation, e.g. a three- or four-phase preparation, substantially as disclosed in, e.g., EP 148 724.

In suitable embodiments of the present preparation, the number of daily dosage units comprising the combination of drospirenone and ethinylestradiol may be 21, 22, 23 or 24, and the number of daily dosage units containing ethinylestradiol alone may then be 7, 6, 5 or 4, as the case may be.

In a further embodiment the present invention relates to the use of drospirenone combined with ethinylestradiol for preparing a pharmaceutical composition for the inhibition of ovulation in a mammal, in particular a human, the composition comprising an amount of drospirenone corresponding to a daily dosage, on administration of the composition, of from about 2 mg to 4 mg, and comprising an amount of ethinylestradiol corresponding to a daily dosage, on administration of the composition, of from about 0.01 to 0.05 mg, wherein at least 70% of said drospirenone is dissolved from said composition within 30 minutes, as determined by USP XXIII Paddle Method II using water at 37°C as the dissolution media and 50 rpm as the stirring rate.

In suitable embodiments, the daily dosage units comprising the combination of drospirenone and ethinylestradiol may be administered for 21, 22, 23 or 24 consecutive days, and the daily dosage units containing no active agent may then be administered for 7, 6, 5 or 4 consecutive days, as appropriate. Furthermore, the daily dosage units comprising the combination of drospirenone and ethinylestradiol may be administered for 28 consecutive days. In a variant of this embodiment, the daily dosage units comprising the combination of drospirenone and ethinylestradiol are administered for 2-4, preferably 2 or 3, times 28 consecutive days, followed by administration of the daily dosage units comprising the combination of drospirenone and ethinylestradiol for 21, 22, 23 or 24 consecutive days and subsequently administration of the daily dosage units containing no active agent, or administration of no daily dosage units, for 7, 6, 5 or 4 consecutive days.

Alternatively, may be administered on each day of at least 21 a daily dosage unit comprising a combination of drospirenone in an amount of from 2 mg to 4 mg and ethinylestradiol in an amount from 0.01 to 0.05 mg consecutive days followed by administering, on each day of 7 or less consecutive days, a daily dosage unit containing ethinylestradiol alone in an amount of from 0.01 mg to 0.05 mg.

In this alternative embodiment, the daily dosage units comprising the combination of drospirenone and ethinylestradiol may suitably be administered for 21, 22, 23 or 24 consecutive days, and wherein the daily dosage units comprising ethinylestradiol alone may then be administered for 7, 6, 5 or 4 consecutive days, as appropriate. In a further embodiment, the daily dosage units comprising the combination of drospirenone and ethinylestradiol are administered for 2-4, preferably 2 or 3, times 28 consecutive days, followed by administration of the daily dosage units comprising the combination of drospirenone and ethinylestradiol for 21 consecutive days and subsequently administration of the daily dosage units comprising ethinylestradiol alone for 7 consecutive days.

For use according to the invention, the pharmaceutical preparation may suitably be in the form of a number of separately packaged and individually removable daily dosage units placed in a packaging unit and intended for oral administration for a period of at least 21 consecutive days, wherein each of said daily dosage units each comprises a combination of drospirenone in an amount of from about 2 mg to about 4 mg and ethinylestradiol in an amount from about 0.01 to about 0.05 mg.

As indicated above, the preparation may further comprise 7 or less daily dosage units containing no active agent (or may contain 7 or less empty "places", e.g. in the form of empty blisters in a blister pack, marking the days on which no daily dosage units are administered).

Alternatively, the pharmaceutical preparation may be in the form of a number of separately packaged and individually removable daily dosage units placed in a packaging unit and intended for oral administration for a period of at least 28 consecutive days, wherein at least 21 of said daily dosage units each comprises a combination of drospirenone in an amount of from 2 mg to 4 mg and ethinylestradiol in an amount of from 0.01 to 0.05 mg, said packaging unit further comprising 7 or less daily dosage units comprising ethinylestradiol alone in an amount of from 0.01 mg to 0.05 mg.

The composition of the invention may be formulated in any manner known in the pharmaceutical art. In particular, as indicated above, the composition may be formulated by a method comprising providing drospirenone and, if desired, ethinylestradiol in micronized form in said unit dosage form, or sprayed from a solution onto particles of an inert carrier in admixture with one or more pharmaceutically acceptable excipients that promote dissolution of the drospirenone and ethinylestradiol so as to promote rapid dissolution of drospirenone and preferably ethinylestradiol on oral administration. Examples of suitable excipients include fillers, e.g. sugars such as lactose, glucose or sucrose, sugar alcohols such as mannitol, sorbitol or xylitol, starch such as wheat, corn or potato starch, modified starch or sodium starch glycolate, lubricants such as talc, magnesium stearate, calcium stearate, colloidal silica or stearic acid, and binders such as polyvinylpyrrolidone, cellulose derivatives, carboxymethyl cellulose, hydroxypropyl cellulose, hydroxypropylmethyl cellulose, methyl cellulose or gelatin, for making oral dosage forms such as tablets, pills or capsules.

Tablets may conveniently be coated with a suitable film-forming agent, e.g. hydroxypropylmethyl cellulose, hydroxypropyl cellulose or ethyl cellulose, to which a suitable excipient may optionally be added, e.g. a softener such as glycerol, propylene glycol, diethylphthalate or glycerol triacetate, filler such as sucrose, sorbitol, xylitol, glucose or lactose, a colorant such as titanium hydroxide, etc.

The present composition may also be formulated in liquid form, e.g. as a solution, suspension or emulsion, together with conventional diluents or excipients in a manner known per se in the pharmaceutical art.

A packaging unit comprising the daily dosage units described above may be prepared in a manner analogous to that of making other oral contraceptives. This may for instance be a conventional blister pack or any other form known for this purpose, for instance a pack comprising the appropriate number of dosage units (in this case at least 21, or for particular applications, 28 or a multiple of 28) in a sealed blister pack with a cardboard, paperboard, foil or plastic backing and enclosed in a suitable cover. Each blister container may conveniently be numbered or otherwise marked, e.g. starting with the first of the at least 21 dosage units that contain the combination of drospirenone and ethinylestradiol, optionally followed by 7 or less empty blisters or by the 7 or less dosage units that contain no active agent or that only contain ethinylestradiol (although the numbering may also start with the first of the 7 or less dosage units that only contain ethinylestradiol).

The present invention is further described in the following examples which are not in any way intended to limit the scope of the invention as claimed.

### EXPERIMENTAL

### Example 1

### Preparation of tablets containing drospirenone and ethinylestradiol

| Tablet cores of the following composition | |
|---|---|
| micronized drospirenone | 3.00 mg |
| micronized ethinylestradiol | 0.03 mg |
| lactose monohydrate | 48.17 mg |
| corn starch | 14.40 mg |
| modified starch | 9.60 mg |
| polyvinylpyrrolidone 25,000 | 4.00 mg |
| magnesium stearate | 0.80 mg |

were prepared by charging a fluidized bed granulator with 31.68 kg corn starch, 21.12 kg modified starch, 6.60 micronized drospirenone, 0.066 kg micronized ethinylestradiol and 105.974 kg lactose monohydrate and activating the fluidized bed. An aqueous solution of 8.80 kg polyvinylpyrrolidone 25,000 in 46.20 kg purified water was sprayed continuously onto the fluidized bed while drying by heating the air stream of the fluidized bed. At the end of the process 1.76 kg magnesium stearate was sucked into the granulator and mixed with the granules by maintaining the fluidized bed. The resulting granulate was pressed into tablet cores by compression using a rotary tablet press.

2.22464 kg of hydroxypropylmethylcellulose and 0.44528 macrogol 6000 were dissolved in 14.67 kg purified water. 0.44528 kg talc, 1.22430 kg titanium dioxide and 0.06050 kg ferric oxide pigment were suspended in 10.26 kg purified water with stirring and homogenized. The solution and suspension were combined and used to coat the tablet cores by continuous application of the coating suspension in a coater.

### Example 2

### Dissolution of drospirenone from tablets

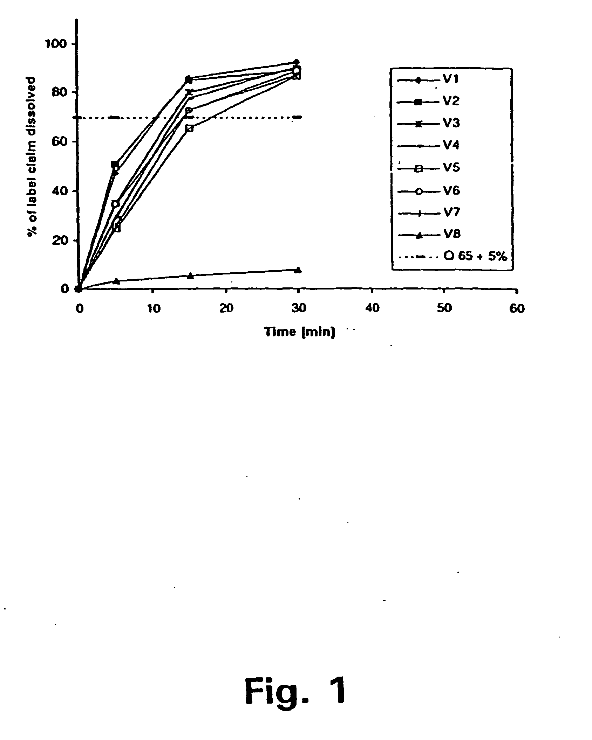

The rate of dissolution of drospirenone from the tablets prepared in Example 1 was determined by the USP XXIII Paddle Method using a USP Dissolution Test Apparatus 2 including 6 covered glass vessels and 6 paddles. Tablets were placed in 900 ml water at a temperature of 37°C (± 0.5°C) and stirred at 50 rpm.
The results appear from Figs. 1, 2 and 4. From Fig. 1, it appears that the batch numbered V8 containing macrocrystalline drospirenone (but otherwise identical to the tablets prepared in Example 1) exhibited an extremely slow dissolution rate of drospirenone, whereas all batches containing micronized drospirenone exhibited a dissolution rate of more than 70% within 30 minutes.

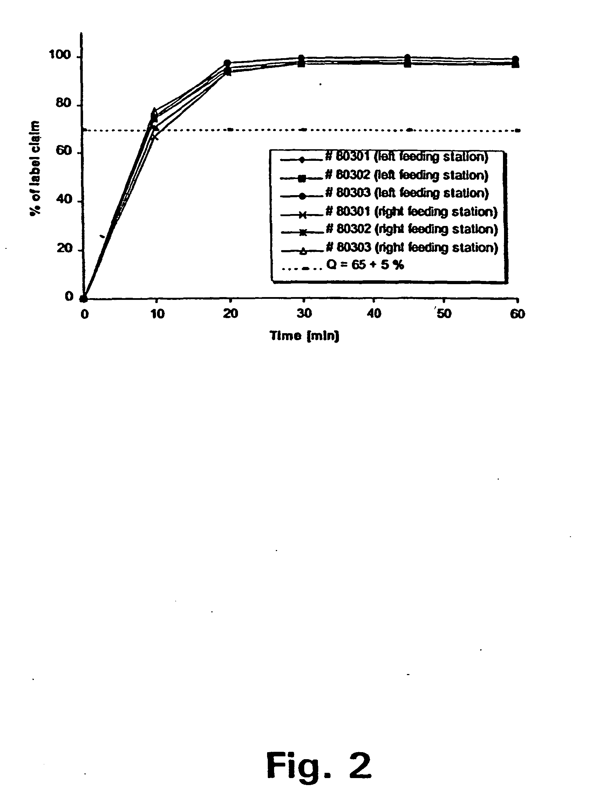

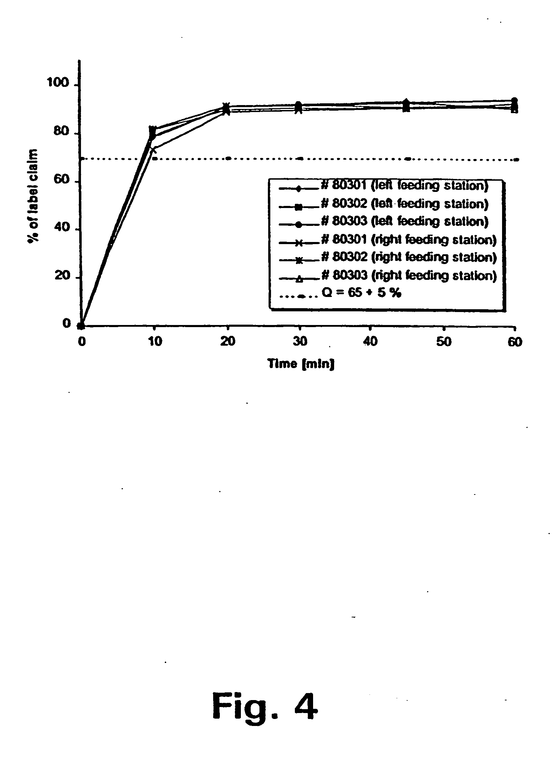

Fig. 2 and Fig. 4 shows the results of dissolution of drospirenone from tablet cores and film-coated tablets, respectively. In both cases more than 70% of the active agent is dissolved within 30 minutes. Thus, the film coating did not significantly influence the rate of dissolution.

### Example 3

### Dissolution rate of ethinylestradiol from tablets in vitro

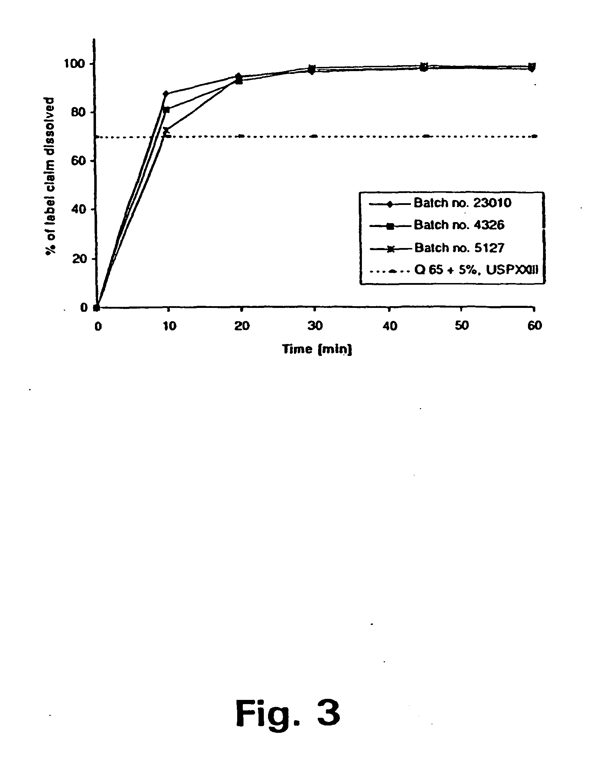

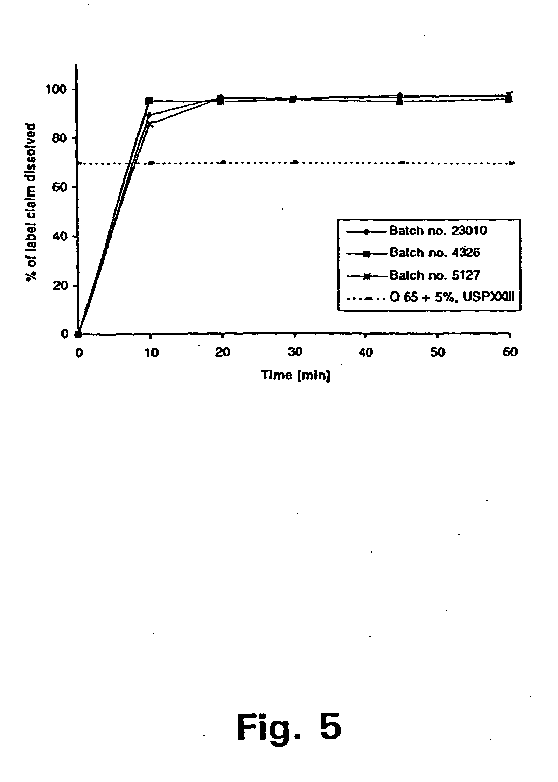

The rate of dissolution of ethinylestradiol from tablets prepared as described in Example 1 was determined according to the USP Paddle Method as described in Example 2 for drospirenone. The results appear from Figs. 3 and 5 showing the dissolution rates from tablet cores and film-coated tablets, respectively. In both cases, more than 70% of the active agent was dissolved within 30 minutes. Thus, the film coating did not significantly influence the rate of dissolution.

### Example 4

### Bioavailability of drospirenone and ethinylestradiol from tablets containing 3 mg of drospirenone and 0.03 mg ethinylestradiol

42 healthy women between 18 and 35 years of age were included in an open-label crossover study after their informed consent had been obtained. The aim of the study was to investigate the relative bioavailability of drospirenone and ethinylestradiol from a tablet formulation containing 3 mg drospirenone and 0.03 mg ethinylestradiol with reference to an oral suspension containing 6 mg of drospirenone and 0.06 mg ethinylestradiol per vial.

The bioavailability was determined using serum concentrations of each active agent as parameters. Compared to the oral suspension, the relative bioavailability of drospirenone and ethinylestradiol from the tablets is 107% and 117%, respectively. It was therefore concluded that both drospirenone and ethinylestradiol are completely released from the tablets in vivo.

The absolute bioavailability of drospirenone was determined in two studies to be 76%±13% after oral administration of 2 mg drospirenone to 8 young healthy women and 85%±24% after oral administration of a microcrystalline suspension containing 3.13 mg drospirenone to 6 postmenopausal women.

The oral bioavaliability of ethinylestradiol was determined in several studies, and mean values of from 36% to 59% were reported in the literature, indicating a first-pass effect.

### Example 5

### Contraceptive efficacy of formulations containing drospirenone and ethinylestradiol

An open-label, randomized trial with 52 female volunteers aged 20-35 years whose informed consent had been obtained included 1 pre-treatment cycle, 3 treatment cycles with two different tablet containing 2 mg and 3 mg drospirenone, respectively, but otherwise corresponding to the tablets prepared in Example 1, and a follow-up phase. A wash-out phase of 1 month preceded the treatment.

At defined time points, selected central and peripheral parameters were investigated: LH, FSH, 17β-estradiol, progesterone, cervical score, "spinnbarkeit", fern phenomenon. Ovarian function was checked by ultrasound. In addition, SHBG, CBG, prolactine, total testosterone, androstenedione, DHEA-S and selected metabolic parameters (serum glucose, triglycerides, cholesterol, HDL, LDL) were examined. Blood pressure, heart rate, body weight and cycle control were documented.

The results of the study showed that both LH and FSH were clearly suppressed with both trial preparations. Accordingly, the secretion of estradiol and progesterone were greatly reduced over all three treatment cycles with the exception of 3 volunteers receiving the 2 mg drospirenone preparation. This result was, in principle, confirmed by the accompanying ultrasound examinations. Follicular ripening occurred in several cases with both trial preparations. Although three ovulations were diagnosed with the preparation containing 2 mg drospirenone (one of which was described as "equivocal" and the other as a "tablet-taking error"), no differences were demonstrable statistically (p > 0.05) between the two trial preparations as regards the hormones LH, FSH, estradiol and progesterone, and the parameter "ovulation during the treatment cycles". In keeping with the hormones, cervical function was greatly limited and the "spinnbarkeit" and crystallisability of the cervical mucus was greatly reduced with both trial preparations. Prolactin increased minimally and SHBG and CBG distinctly with both preparations. Triglycerides and HDL levels increased with both trial preparations, while LDL levels decreased. Total cholesterol was largely unchanged in both treatment groups. Oral glucose tolerance remained virtually unchanged or was slightly decreased. Testosterone, androstenedione and DHEA-S decreased minimally.

The subjective and objective tolerance was good with both treatments. This was also the case for cycle control with the exception of the first cycle with 2 mg drospirenone. Blood pressure, heart rate and body weight remained constant in the majority of cases or showed a slight tendency to decrease.

### After three months' treatment, it was concluded:

The two trial preparations were equally good as regards the subjective and objective tolerance.

No negative metabolic effects were observed with either preparation. HDL was influenced positively in the sense of an increase.
The results confirmed the results of earlier studies that the 2 mg drospirenone preparation was in the threshold region of ovulation inhibition, whereas the 3 mg drospirenone preparation had a demonstrable ovulation-inhibiting effect in all cases examined.

## Claims

1. A pharmaceutical composition comprising, as a first active agent drospirenone in an amount corresponding to a daily dosage, on administration of the composition, of from about 2 mg to 4 mg, and as a second active agent, ethinylestradiol in an amount corresponding to a daily dosage of from about 0.01 mg to 0.05 mg, together with one or more pharmaceutically acceptable carriers or excipients, wherein at least 70% of said drospirenone is dissolved from said composition within 30 minutes, as determined by USP XXIII Paddle Method II using water at 37°C as the dissolution media and 50 rpm as the stirring rate.

2. The composition according to claim 1 comprising drospirenone in an amount corresponding to a daily dosage of from 2.5 mg to 3.5 mg.

3. The composition according to claim 2 comprising drospirenone in an amount corresponding to a daily dosage of about 3 mg.

4. The composition according to any of claims 1 to 3 comprising ethinylestradiol in an amount corresponding to a daily dosage of from 0.015 mg to 0.04 mg.

5. The composition according to claim 4 comprising ethinylestradiol in an amount corresponding to a daily dosage of from 0.015 mg to 0.03 mg.

6. The composition according to claim 1 comprising drospirenone in an amount corresponding to a daily dosage of from 3.0 mg to 3.5 mg, and ethinylestradiol in an amount corresponding to a daily dosage of from 0.015 mg to 0.03 mg.

7. The composition according to any of claims 1 to 6, wherein drospirenone is sprayed from a solution of drospirenone onto particles of an inert carrier.

8. The composition according to any of claims 1 to 7, wherein the in vitro dissolution of ethinylestradiol is such that at least 70% is dissolved within 30 minutes, as determined by USP XXIII Paddle Method II using water at 37°C as the dissolution media and 50 rpm as the stirring rate.

9. The composition according to any of claims 1 to 8, wherein ethinylestradiol is in micronized form.

10. The composition according to any of claims 1 to 9, wherein said composition is in the form of an oral dosage form.

11. The composition according to claim 10, wherein said oral dosage form is a tablet, pill or capsule.

12. A pharmaceutical preparation consisting of a number of separately packaged and individually removable daily dosage units placed in a packaging unit and intended for oral administration for a period of at least 21 consecutive days, wherein said daily dosage units comprises a combination of drospirenone in an amount of from about 2 mg to 4 mg and ethinylestradiol in an amount from about 0.01 to 0.05 mg, wherein at least 70% of said drospirenone is dissolved from said dosage unit within 30 minutes, as determined by USP XXIII Paddle Method II using water at 37°C as the dissolution media and 50 rpm as the _ stirring rate.

13. The preparation according to claim 12, wherein the in vitro dissolution of ethinylestradiol is such that at least 70% is dissolved from said dosage unit within 30 minutes, as determined by USP XXIII Paddle Method II using water at 37°C as the dissolution media and 50 rpm as the stirring rate.

14. The preparation according to claim 12 or 13, wherein drospirenone is sprayed from a solution of drospirenone onto particles of an inert carrier.

15. The preparation according to any of claims 12 to 14, which additionally comprises 7 or less daily dosage units containing no active agent intended for oral administration subsequent to the period of at least 21 consecutive days, the total number of daily dosage units being at least 28.

16. The preparation according to any of claims 12 to 14, wherein the number of daily dosage units comprising the combination of drospirenone and ethinylestradiol is 21, 22, 23 or 24, and wherein the number of daily dosage units containing no active agent is 7, 6, 5 or 4.

17. The preparation according to any of claims 12 to 14 intended for oral administration for a period of at least 28 consecutive days, wherein at least 21 of said daily dosage units comprises a combination of drospirenone in an amount of from about 2 mg to 4 mg and ethinylestradiol in an amount from about 0.01 to 0.05 mg, and wherein 7 or less of said daily dosage units contain ethinylestradiol alone in an amount from about 0.01 to 0.05 mg.

18. The preparation according to any of claims 12 to 14, wherein the number of daily dosage units comprising the combination of drospirenone and ethinylestradiol is 21, 22, 23 or 24, and wherein the number of daily dosage units comprising ethinylestradiol alone is 7, 6, 5 or 4.

19. The preparation according to any of claims 12 to 15, wherein the number of daily dosage units comprising the combination of drospirenone and ethinylestradiol is 28, or a multiple of 28 such as 2-4, in particular 2 or 3, times 28.

20. The preparation according to claim 12, wherein the number of daily dosage units intended for oral administration is for 2-4, preferably 2 or 3, times 28 consecutive days, followed by administration of the daily dosage units comprising the combination of drospirenone and ethinylestradiol for 21, 22, 23 or 24 consecutive days and subsequently administration of the daily dosage units containing no active agent, or administration of no daily dosage units, for 7, 6, 5 or 4 consecutive days.

21. Use of drospirenone combined with ethinylestradiol for preparing a pharmaceutical composition for the inhibition of ovulation in a mammal, in particular a human, the composition comprising an amount of drospirenone corresponding to a daily dosage, on administration of the composition, of from about 2 mg to 4 mg, and comprising an amount of ethinylestradiol corresponding to a daily dosage, on administration of the composition, of from about 0.01 to 0.05 mg, wherein at least 70% of said drospirenone is dissolved from said composition within 30 minutes, as determined by USP XXIII Paddle Method II using water at 37°C as the dissolution media and 50 rpm as the stirring rate.

22. The use according to claim 21, wherein drospirenone is sprayed from a solution of drospirenone onto particles of an inert carrier.

23. The use according to claim 21 or 22, wherein at least 70% of ethinyl estradiol is dissolved from the composition within 30 minutes, as determined by USP XXIII Paddle Method II using water at 37°C as the dissolution media and 50 rpm as the stirring rate.

## Patentansprüche

1. Pharmazeutische Zusammensetzung, enthaltend als ersten Wirkstoff Drospirenon in einer Menge, die bei Verabreichung der Zusammensetzung einer Tagesdosis von etwa 2 mg bis 4 mg entspricht, und als zweiten Wirkstoff Ethinylestradiol in einer Menge, die einer Tagesdosis von etwa 0,01 mg bis 0,05 mg entspricht, zusammen mit einem oder mehreren pharmazeutisch verträglichen Trägern oder Hilfsstoffen, wobei gemäß Bestimmung nach der Paddle-Methode II gemäß USP XXIII unter Verwendung von Wasser bei 37°C als Auflösungsmedium und 50 U/min als Rührgeschwindigkeit innerhalb von 30 Minuten mindestens 70% des Drospirenons aus der Zusammensetzung herausgelöst werden.

2. Zusammensetzung nach Anspruch 1, enthaltend Drospirenon in einer Menge, die einer Tagesdosis von 2,5 mg bis 3,5 mg entspricht.

3. Zusammensetzung nach Anspruch 2, enthaltend Drospirenon in einer Menge, die einer Tagesdosis von etwa 3 mg entspricht.

4. Zusammensetzung nach einem der Ansprüche 1 bis 3, enthaltend Ethinylestradiol in einer Menge, die einer Tagesdosis von 0,015 mg bis 0,04 mg entspricht.

5. Zusammensetzung nach Anspruch 4, enthaltend Ethinylestradiol in einer Menge, die einer Tagesdosis von 0,015 mg bis 0,03 mg entspricht.

6. Zusammensetzung nach Anspruch 1, enthaltend Drospirenon in einer Menge, die einer Tagesdosis von 3,0 mg bis 3,5 mg entspricht, und Ethinylestradiol in einer Menge, die einer Tagesdosis von 0,015 mg bis 0,03 mg entspricht.

7. Zusammensetzung nach einem der Ansprüche 1 bis 6, wobei Drospirenon aus einer Lösung von Drospirenon auf Teilchen eines inerten Trägers aufgesprüht worden ist.

8. Zusammensetzung nach einem der Ansprüche 1 bis 7, wobei die in-vitro-Auflösung des Ethinylestradiols so beschaffen ist, daß gemäß Bestimmung nach der Paddle-Methode II gemäß USP XXIII unter Verwendung von Wasser bei 37°C als Auflösungsmedium und 50 U/min als Rührgeschwindigkeit innerhalb von 30 Minuten mindestens 70% gelöst werden.

9. Zusammensetzung nach einem der Ansprüche 1 bis 8, wobei Ethinylestradiol in mikronisierter Form vorliegt.

10. Zusammensetzung nach einem der Ansprüche 1 bis 9, die in Form einer oralen Dosisform vorliegt.

11. Zusammensetzung nach Anspruch 10, wobei es sich bei der oralen Dosisform um eine Tablette, Pille oder Kapsel handelt.

12. Pharmazeutische Zubereitung, bestehend aus einer Zahl getrennt verpackter und einzeln herausnehmbarer Tagesdosiseinheiten, die in einer Verpackungseinheit angeordnet und für die orale Verabreichung über einen Zeitraum von mindestens 21 aufeinanderfolgenden Tagen vorgesehen sind, wobei die Tagesdosiseinheiten eine Kombination von Drospirenon in einer Menge von etwa 2 mg bis 4 mg und Ethinylestradiol in einer Menge von etwa 0,01 bis 0,05 mg enthalten, wobei gemäß Bestimmung nach der Paddle-Methode II gemäß USP XXIII unter Verwendung von Wasser bei 37°C als Auflösungsmedium und 50 U/min als Rührgeschwindigkeit innerhalb von 30 Minuten mindestens 70% des Drospirenons aus der Dosiseinheit herausgelöst werden.

13. Zubereitung nach Anspruch 12, wobei die in-vitro-Auflösung des Ethinylestradiols so beschaffen ist, daß gemäß Bestimmung nach der Paddle-Methode II gemäß USP XXIII unter Verwendung von Wasser bei 37°C als Auflösungsmedium und 50 U/min als Rührgeschwindigkeit innerhalb von 30 Minuten mindestens 70% aus der Dosiseinheit gelöst werden.

14. Zubereitung nach Anspruch 12 oder 13, wobei Drospirenon aus einer Lösung von Drospirenon auf Teilchen eines inerten Trägers aufgesprüht worden ist.

15. Zubereitung nach einem der Ansprüche 12 bis 14, die zusätzlich 7 oder weniger Tagesdosiseinheiten, die keinen Wirkstoff enthalten und für die orale Verabreichung nach dem Zeitraum von mindestens 21 aufeinanderfolgenden Tagen bestimmt sind, enthält, wobei die Gesamtzahl der Tagesdosiseinheiten mindestens 28 beträgt.

16. Zubereitung nach einem der Ansprüche 12 bis 14, wobei die Zahl der Tagesdosiseinheiten, die die Kombination von Drospirenon und Ethinylestradiol enthalten, 21, 22, 23 oder 24 beträgt, und die Zahl der Tagesdosiseinheiten, die keinen Wirkstoff enthalten, 7, 6, 5 oder 4 beträgt.

17. Zubereitung nach einem der Ansprüche 12 bis 14, die zur oralen Verabreichung über einen Zeitraum von mindestens 28 aufeinanderfolgenden Tagen vorgesehen ist, wobei mindestens 21 der Tagesdosiseinheiten eine Kombination von Drospirenon in einer Menge von etwa 2 mg bis 4 mg und Ethinylestradiol in einer Menge von etwa 0,01 bis 0,05 mg enthalten und 7 oder weniger der Tagesdosiseinheiten nur Ethinylestradiol in einer Menge von etwa 0,01 bis 0,05 mg enthalten.

18. Zubereitung nach einem der Ansprüche 12 bis 14, wobei die Zahl der Tagesdosiseinheiten, die die Kombination von Drospirenon und Ethinylestradiol enthalten, 21, 22, 23 oder 24 beträgt, und die Zahl der Tagesdosiseinheiten, die nur Ethinylestradiol enthalten, 7, 6, 5 oder 4 beträgt.

19. Zubereitung nach einem der Ansprüche 12 bis 15, wobei die Zahl der Tagesdosiseinheiten, die die Kombination von Drospirenon und Ethinylestradiol enthalten, 28 oder ein Vielfaches von 28, wie 2-4mal und insbesondere 2- oder 3mal 28, beträgt.

20. Zubereitung nach Anspruch 12, wobei die Zahl der für die orale Verabreichung vorgesehenen Tagesdosiseinheiten für 2-4mal und vorzugsweise 2- oder 3mal 28 aufeinanderfolgende Tage ist, gefolgt von der Verabreichung der Tagesdosiseinheiten, die die Kombination von Drospirenon und Ethinylestradiol enthalten, über einen Zeitraum von 21, 22, 23 oder 24 aufeinanderfolgenden Tagen und danach Verabreichung der Tagesdosiseinheiten, die keinen Wirkstoff enthalten, oder Verabreichung keiner Tagesdosiseinheiten über einen Zeitraum von 7, 6, 5 oder 4 aufeinaderfolgenden Tagen.

21. Verwendung von Drospirenon in Kombination mit Ethinylestradiol zur Herstellung einer pharmazeutischen Zusammensetzung zur Inhibierung der Ovulation bei einem Säugetier, insbesondere einem Menschen, wobei die Zusammensetzung eine Drospirenon-Menge, die bei Verabreichung der Zusammensetzung einer Tagesdosis von etwa 2 mg bis 4 mg entspricht, und eine Ethinylestradiol-Menge, die bei Verabreichung der Zusammensetzung einer Tagesdosis von etwa 0,01 mg bis 0,05 mg entspricht, wobei gemäß Bestimmung nach der Paddle-Methode II gemäß USP XXIII unter Verwendung von Wasser bei 37°C als Auflösungsmedium und 50 U/min als Rührgeschwindigkeit innerhalb von 30 Minuten mindestens 70% des Drospirenons aus der Zusammensetzung herausgelöst werden.

22. Verwendung nach Anspruch 21, wobei Drospirenon aus einer Lösung von Drospirenon auf Teilchen eines inerten Trägers aufgesprüht worden ist.

23. Verwendung nach Anspruch 21 oder 22, wobei gemäß Bestimmung nach der Paddle-Methode II gemäß USP XXIII unter Verwendung von Wasser bei 37°C als Auflösungsmedium und 50 U/min als Rührgeschwindigkeit innerhalb von 30 Minuten mindestens 70% Ethinylestradiol aus der Zusammensetzung herausgelöst werden.

## Revendications

1. Composition pharmaceutique comprenant, comme premier agent actif, de la drospirénone en une quantité correspondant à un dosage journalier, lors de l'administration de la composition, allant d'environ 2 mg à 4 mg, et comme deuxième agent actif, de l'éthinylestradiol en une quantité correspondant à un dosage journalier allant d'environ 0,01 mg à 0,05 mg, conjointement avec un ou plusieurs véhicules ou excipients pharmaceutiquement acceptables, dans laquelle au moins 70% de ladite drospirénone est solubilisée à partir de ladite composition dans les 30 minutes, tel que déterminé par une Méthode à Pales II USP XXIII en utilisant de l'eau à 37°C comme milieu de solubilisation et 50 tours/min comme vitesse d'agitation.

2. Composition selon la revendication 1, comprenant de la drospirénone en une quantité correspondant à un dosage journalier allant de 2,5 mg à 3,5 mg.

3. Composition selon la revendication 2, comprenant de la drospirénone en une quantité correspondant à un dosage journalier d'environ 3 mg.

4. Composition selon l'une quelconque des revendications 1 à 3, comprenant de l'éthinylestradiol en une quantité correspondant à un dosage journalier allant de 0,015 mg à 0,04 mg.

5. Composition selon la revendication 4, comprenant de l'éthinylestradiol en une quantité correspondant à un dosage journalier allant de 0,015 mg à 0,03 mg.

6. Composition selon la revendication 1, comprenant de la drospirénone en une quantité correspondant à un dosage journalier allant de 3,0 mg à 3,5 mg, et de l'éthinylestradiol en une quantité correspondant à un dosage journalier allant de 0,015 mg à 0,03 mg.

7. Composition selon l'une quelconque des revendications 1 à 6, dans laquelle la drospirénone est pulvérisée à partir d'une solution de drospirénone sur des particules d'un véhicule inerte.

8. Composition selon l'une quelconque des revendications 1 à 7, dans laquelle la solubilisation *in vitro* de l'éthinylestradiol est telle qu'au moins 70% est solubilisé dans les 30 minutes, tel que déterminé par une Méthode à Pales II USP XXIII en utilisant de l'eau à 37°C comme milieu de solubilisation et 50 tours/min comme vitesse d'agitation.

9. Composition selon l'une quelconque des revendications 1 à 8, dans laquelle l'éthinylestradiol est sous forme micronisée.

10. Composition selon l'une quelconque des revendications 1 à 9, dans laquelle ladite composition est sous la forme d'une forme de dosage orale.

11. Composition selon la revendication 10, dans laquelle ladite forme de dosage orale est un comprimé, une pilule ou une capsule.

12. Préparation pharmaceutique constituée d'un certain nombre d'unités de dosage journalier conditionnées séparément et extractibles individuellement placées dans une unité de conditionnement et prévues pour une administration par voie orale pendant une période d'au moins 21 jours consécutifs, dans laquelle lesdites unités de dosage journalier comprennent une association de drospirénone en une quantité allant d'environ 2 mg à 4 mg et de l'éthinylestradiol en une quantité allant d'environ 0,01 à 0,05 mg, dans laquelle au moins 70% de ladite drospirénone est solubilisée à partir de ladite unité de dosage dans les 30 minutes, tel que déterminé par une Méthode à Pales II USP XXIII en utilisant de l'eau à 37°C comme milieu de solubilisation et 50 tours/min comme vitesse d'agitation.

13. Préparation selon la revendication 12, dans laquelle la solubilisation *in vitro* de l'éthinyl-estradiol est telle qu'au moins 70% est solubilisé à partir de ladite unité de dosage dans les 30 minutes, tel que déterminé par une Méthode à Pales II USP XXIII en utilisant de l'eau à 37°C comme milieu de solubilisation et 50 tours/min comme vitesse d'agitation.

14. Préparation selon la revendication 12 ou 13, dans laquelle la drospirénone est pulvérisée à partir d'une solution de drospirénone sur des particules d'un véhicule inerte.

15. Préparation selon l'une quelconque des revendications 12 à 14, comprenant en outre 7 ou moins unités de dosage journalier ne contenant aucun agent actif prévues pour une administration par voie orale consécutive à la période d'au moins 21 jours consécutifs, le nombre total d'unités de dosage journalier étant d'au moins 28.

16. Préparation selon l'une quelconque des revendications 12 à 14, dans laquelle le nombre d'unités de dosage journalier comprenant l'association de drospirénone et d'éthinylestradiol est de 21, 22, 23 ou 24, et dans laquelle le nombre d'unités de dosage journalier ne contenant aucun agent actif est de 7, 6, 5 ou 4.

17. Préparation selon l'une quelconque des revendications 12 à 14, prévue pour une administration par voie orale pendant une période d'au moins 28 jours consécutifs, dans laquelle au moins 21 desdites unités de dosage journalier comprennent une association de drospirénone en une quantité allant d'environ 2 mg à 4 mg et de l'éthinylestradiol en une quantité allant d'environ 0,01 à 0,05 mg, et dans laquelle 7 ou moins desdites unités de dosage journalier contiennent de l'éthinylestradiol seul en une quantité allant d'environ 0,01 à 0,05 mg.

18. Préparation selon l'une quelconque des revendications 12 à 14, dans laquelle le nombre d'unités de dosage journalier comprenant l'association de drospirénone et d'éthinylestradiol est de 21, 22, 23 ou 24, et dans laquelle le nombre d'unités de dosage journalier comprenant de l'éthinylestradiol seul est de 7, 6, 5 ou 4.

19. Préparation selon l'une quelconque des revendications 12 à 15, dans laquelle le nombre d'unités de dosage journalier comprenant l'association de drospirénone et d'éthinylestradiol est de 28, ou un multiple de 28 tel que 2-4, en particulier 2 ou 3, fois 28.

20. Préparation selon la revendication 12, dans laquelle le nombre d'unités de dosage journalier prévues pour une administration par voie orale est pour 2-4, préférablement 2 ou 3, fois 28 jours consécutifs, suivis de l'administration des unités de dosage journalier comprenant l'association de drospirénone et d'éthinylestradiol pendant 21, 22, 23 ou 24 jours consécutifs, puis l'administration des unités de dosage journalier ne contenant aucun agent actif, ou l'administration d'aucune unité de dosage journalier pendant 7, 6, 5 ou 4 jours consécutifs.

21. Utilisation de drospirénone en association avec de l'éthinylestradiol pour la préparation d'une composition pharmaceutique destinée à l'inhibition de l'ovulation chez un mammifère, en particulier un être humain, la composition comprenant une quantité de drospirénone correspondant à un dosage journalier, lors de l'administration de la composition, allant d'environ 2 mg à 4 mg, et comprenant une quantité d'éthinyl-estradiol correspondant à un dosage journalier, lors de l'administration de la composition, allant d'environ 0,01 à 0,05 mg, dans laquelle au moins 70% de ladite drospirénone est solubilisée à partir de ladite composition dans les 30 minutes, tel que déterminé par une Méthode à Pales II USP XXIII en utilisant de l'eau à 37°C comme milieu de solubilisation et 50 tours/min comme vitesse d'agitation.

22. Utilisation selon la revendication 21, dans laquelle la drospirénone est pulvérisée à partir d'une solution de drospirénone sur des particules d'un véhicule inerte.

23. Utilisation selon la revendication 21 ou 22, dans laquelle au moins 70% d'éthinylestradiol est solubilisé à partir de la composition dans les 30 minutes, tel que déterminé par une Méthode à Pales II USP XXIII en utilisant de l'eau à 37°C comme milieu de solubilisation et 50 tours/min comme vitesse d'agitation.
